# EUROPÄISCHE PATENTANMELDUNG

(11) **EP 1 921 552 A1**
(43) Veröffentlichungstag der Anmeldung: **14.05.2008**
(21) Anmeldenummer: 06023552.0
(22) Anmeldetag: 13.11.2006
(51) Int. Cl.: G06F 19/00

(54) **Netzplangesteuertes Verfahren zur Sicherstellung der Authentizität und Qualität visuell erhobener Befunde in der Indirekten Immunfluoreszenz**

(71) Anmelder: Euroimmun Medizinische Labordiagnostika AG, 23560 Lübeck (DE)
(72) Erfinder: Stöcker, Dr. Winfried, 23627 Gross Grönau (DE); Voigt, Dr. Jörn Frederik, 23558 Lübeck (DE); Berg, Dr. Sabine, 23560 Lübeck (DE); Fauer, Hendrik, 23617 Stockelsdorf (DE); Roznowicz, Rafael, 23562 Lübeck (DE)
(74) Vertreter: UEXKÜLL & STOLBERG

(57) **Zusammenfassung**

Die Erfindung betrifft ein netzplangesteuertes Verfahren, das bei manuell oder teilautomatisiert durchgeführten medizinischen Laboranalysen mit der Technik "Indirekte Immunfluoreszenz" durch eine elektronische Vernetzung der Probenprozessierungsschritte menschliche Fehlerquellen wirksam reduziert und gewährleistet, dass die durch das Laborpersonal visuell erhobenen Einzelbefunde auf einer korrekten und fehlerfrei zur untersuchten Patientenprobe assoziierten Datenbasis beruhen. Die Erfindung verbessert die Authentizität und die Qualität von Befunden, die auf der Basis visueller Analyse histochemischer oder cytochemischer Fluoreszenzbilder erstellt wurden.

## Beschreibung

### Erfindungsgebiet

Die Erfindung betrifft ein netzplangesteuertes Verfahren zur Sicherstellung der Authentizität und Qualität visuell erhobener Befunde in der Labordiagnostik aufgrund manuell oder teilautomatisiert durchgeführter medizinischer Laboranalysen mit der Technik "Indirekte Immunfluoreszenz". Das dabei verwendete Laborsystem reduziert durch eine elektronische Vernetzung der Probenprozessierungsschritte menschliche Fehlerquellen und gewährleistet, dass die visuell erhobenen Befunde auf einer korrekten und fehlerfrei zur untersuchten Patientenprobe assoziierten Datenbasis beruhen.

### Stand der Technik

In der medizinischen Labordiagnostik können über den Nachweis von Antikörpern in Patientenproben Autoimmun- und Infektionskrankheiten sowie Allergien diagnostiziert werden. In der "Indirekten Immunfluoreszenz" (IIF) dienen zum Nachweis dieser Antikörper auf Probenträger aufgetragene Zellen, Gewebeschnitte oder biochemisch definierbare Substanzen. Die Befunde werden nach manueller oder teilautomatisierter Probenprozessierung visuell durch Beurteilung der Fluoreszenzbilder jedes Probenträgerfeldes mittels Fluoreszenz-Mikroskop analysiert und die Ergebnisse manuell erfasst.

Die medizinisch erhobenen Laborbefunde müssen dem Patienten korrekt und fehlerfrei zugeordnet werden. Bei vielen Verfahren der medizinischen Labordiagnostik, wie dem Enzyme-Linked Immunosorbent Assay (ELISA), ist dieses in erster Linie durch eine computergestützte Organisation der Laborarbeitsabläufe von der Erstellung der Probenverdünnungen über die Durchführung der Inkubationen bis hin zur automatisierten Auswertung gelungen, mit denen die "Fehlerquelle Mensch" umgangen wird. Für Teilbereiche der Labordiagnostik, u. a. die "Indirekte Immunfluoreszenz", in denen die Befunde visuell erhoben werden, da es insbesondere noch keine zuverlässige automatisierte Klassifikation von Fluoreszenzbildern gibt, gab es bisher noch keine konsequenten Lösungsansätze, menschliches Versagen und insbesondere die Fehlzuordnung von Befund- zu Patientendaten auszuschließen.

Im Detail bestehen bei der visuellen Befunderhebung die Gefahren, dass inkubierte Probenträger vertauscht werden, Felder auf den Probenträgern verwechselt werden oder es können insbesondere aufgrund der Situation in der Dunkelkammer falsche Zuordnungen von Befund- zu Patientendaten erfolgen, wenn die am Mikroskop abgelesenen Befunde auf einem Protokoll für den falschen Patienten eingetragen werden.

Darüber hinaus wird die Technik "Indirekte Immunfluoreszenz" aufgrund des Fehlens einer möglichen Vollautomatisierung und aufgrund der zum Teil geringen Untersuchungsmengen in einer Vielzahl von Laboren aus Effizienzgründen vollständig manuell durchgeführt. Diese manuellen Verfahren bergen eine Reihe weiterer Fehlerquellen, die zu einer Verfälschung der Befunde führen können, So besteht beispielsweise die Gefahr, dass bei der Herstellung der Verdünnungsstufen Patientenproben verwechselt, einzelne Patientenproben auf falschen Probenträgerfeldern oder mit falschen Reagenzien inkubiert werden.

Dies verdeutlicht, dass in der "Indirekten Immunfluoreszenz" der Qualitätsstandard "Befund-Authentizität" nicht zufrieden stellend gewährleistet ist.

**Aufgabe** der Erfindung ist es, ein netzplangesteuertes Verfahren zu schaffen, das bei manuell oder teilautomatisiert durchgeführten medizinischen Laboranalysen mit der Technik "Indirekte Immunfluoreszenz" durch eine elektronische Vernetzung der Probenprozessierungsschritte menschliche Fehlerquellen wirksam reduziert und gewährleistet, dass die visuell erhobenen Einzelbefunde auf einer korrekten und fehlerfrei zur untersuchten Patientenprobe assoziierten Datenbasis beruhen.

Zur **Lösung** dieser Aufgabe dient das Verfahren nach Patentanspruch 1. Dies ist ein netzplangesteuertes Verfahren zur SiCherstellung der Authentizität und Qualität visuell erhobener Befunde aufgrund manuell oder teilautomatisiert durchgeführter medizinischer Laboranalysen mit der Technik "Indirekte Immunfluoreszenz" mit den Schritten:
a) Elektronisches Erfassen von Daten, die Patientenproben eindeutig identifizieren und elektronisches Zuordnen der Untersuchungsanforderungen;
b) elektronisches Generieren der Vorgaben für Verdünnungsstufen von Patientenproben und der Anforderungslisten für Reagenzien und Probenträger, auf denen Zellen, Gewebeschnitte oder biochemisch definierbare Substanzen aufgetragen sind;
c) elektronisches Generieren der Inkubationspläne, die die Patientenprobendaten den Probenträgerdaten zuordnen und Probendatensätze erzeugen, die hinsichtlich der Patientenproben und Probenträger eindeutig sind und die alle Einwirkungen auf die Patientenprobe vorgeben, so dass zu jeder Zeit abrufbar ist, welchen Prozessen die Patientenprobe zu unterwerfen ist bzw, unterworfen wurde;
d) elektronisches Erfassen der Daten von Probenträgern;
e) elektronisches Identifizieren der inkubierten und eindeutig über eine Identifikationsnummer gekennzeichneten Probenträger ;
f) elektronisches Assoziieren der Befundeingabemaske zur Patientenprobe mit dem über eine Messvorrichtung exakt erfassten fluoreszierenden Probenträgerfeld unter dem Mikroskop;
g) elektronisch gesteuerte Aktualisierung des Probenträgerfeldes unter dem Mikroskop über einen Schrittmotor mit Messvorrichtung, der mit Abschluss der Befundeingabe den Probenträger unter dem Mikroskop zum nächsten Feld fährt;
h) elektronisch ausgelöste Aufnahme des Fluoreszenzbildes eines Probenträgerfeldes, bei dem über einen Shutter im Augenblick der Aufnahme 100% des Mikroskoplichts der Kamera zugeteilt wird;
i) elektronisches Archivieren der Fluoreszenzbilder und Zuordnen zu dem Probendatensatz, so dass zu jeder Zeit der erhobene Befund auf Basis dieser Bilder verifiziert werden kann;
j) elektronisches Eingeben aller visuell ermittelten Befunddaten in der assoziierten Befundeingabemaske ;
k) elektronisches Signieren des Patientenbefundes ; und
l) elektronisches Versenden des Patientenbefundes.

Das zur Durchführung der Erfindung verwendete Laborsystem, bestehend aus einer Datenbank, in der die Logik für die Steuerung der Probenprozessierung abgelegt ist, einer Steuerungssoftware und einem Mikroskoparbeitsplatz mit Schrittmotor, Messvorrichtung und Kamera, realisiert eine elektronische Vernetzung der Probenprozessierungsschritte in der "Indirekten Immunfluoreszenz" zur Erstellung visueller erhobener Patientenbefunde unhabhängig vom Automatisierungsgrad im Labor.

Die Probenprozessierung beginnt mit dem elektronischen Erfassen der Daten, die die Patientenproben eindeutig identifizieren und dem elektronischen Zuordnen der Untersuchungsanforderungen. Vom Laborsystem generiert werden die Vorgaben für die Verdünnungsstufen der Patientenproben, die Anforderungslisten für Probenträger und Reagenzien und die Inkubationspläne. Die Inkubationspläne enthalten vom Laborsystem erzeugte Probendatensätze, die hinsichtlich Patientenproben und Probenträger eindeutig sind und eineindeutig assoziiert sind mit den zu inkubierenden Probenträgerfeldern. Die Inkubationspläne geben alle Einwirkungen vor, denen die Verdünnungen der Patientenproben auf den unterschiedlichen Probenträgerfeldern zu unterziehen sind.

Die Verdünnungsstufen werden gemäß den Vorgaben des Laborsystems erstellt. Die Probenträger und Reagenzien werden dem Lager entsprechend den Anforderungslisten entnommen und die Daten der Probenträger elektronisch im Laborsystem erfasst. Die Inkubationen auf den Probenträgerfeldern werden wie von den Inkubationsplänen vorgegeben durchgeführt. Diese Vorgänge können je nach realisiertem Automatisierungsgrad im Labor manuell gemäß der Vorgaben des Laborsystems vom Laborpersonal oder automatisiert durch vom Laborsystem angesteuerte Automaten durchgeführt werden.

Die visuelle Befunderhebung der Fluoreszenzbilder auf den Probenträgerfeldern wird am Mikroskoparbeitsplatz durchgeführt, der aus einer mit dem Laborsystem verknüpften Anordnung eines Mikroskoptisches mit Schrittmotor, Messvorrichtung und Kamera besteht. Der inkubierte Probenträger wird zur Befunderhebung im Laborsystem elektronisch identifiziert. Die Befundeingabemaske zur Patientenprobe wird elektronisch assoziiert mit dem über eine Messvorrichtung exakt erfassten fluoreszierenden Probenträgerfeld unter dem Mikroskop. Der visuell erhobene Befund wird in der assoziierten Befundeingabemaske zur Patientenprobe erstellt. Die Befunddaten bestehen dabei zum einen aus Ergebnissen zum Probenträgerfeld, die elektronisch korrekt und eineindeutig dem Probendatensatz zugeordnet werden und zum anderen aus individuellen Befundtexten, die elektronisch korrekt und eineindeutig den Patientenprobendaten zugeordnet werden.

Die Qualität der visuellen Befunderhebung am Mikroskop und der Befundverifikation auf Basis der für den jeweiligen Patienten archivierten Fluoreszenzbilder wird dadurch erhöht, dass der Kamera im Augenblick der Aufnahme des Fluoreszenzbildes 100% des Lichts über einen Shutter zugeteilt wird, anstelle der sonst üblichen Teilung des Nichts zwischen Beobachter und Kamera. Das Bild wird archiviert und dem Probendatensatz zugeordnet. Mit Abschluss der Befundeingabe zu einem Probendatensatz fährt der Schrittmotor mit Messvorrichtung den Probenträger unter dem Mikroskop zum nächsten Feld.

Die Erfindung stellt damit sicher, dass die visuell erhobenen Befunddaten elektronisch korrekt und eineindeutig den Patientenprobendaten zugeordnet werden.

Die archivierten authentischen Fluoreszenzbildern werden vom Laborarzt bei der epikritischen Befundverifikation genutzt, um auf deren Basis zusammen mit allen Kontrolluntersuchungen die individuellen Patientenbefunde zu verifizieren, gegebenenfalls zu reevaluieren und anschließend elektronisch zu signieren und zu versenden.

Bei Anwendung des erfindungsgemäßen netzplangesteuerten Laborsystems werden menschliche Fehlerquellen und damit die Gefahr möglicher Falschbefunderhebungen in der "Indirekten Immunfluoreszenz" wirksam reduziert und es wird sichergestellt, dass die durch das Laborpersonal, visuell erhobenen und vom Laborarzt verifizierten Einzelbefunde auf einer korrekten und fehlerfrei zur untersuchten Patientenprobe assoziierten Datenbasis beruhen, und dass die Befunde dem Einsender der Patientenproben und Untersuchungsanforderungen elektronisch zugestellt werden.

Die Erfindung verbessert die Authentizität und die Qualität von Befunden, die auf der Basis visueller Analyse von Fluoreszenzbildern erstellt wurden.

Die Erfindung wird nachfolgend anhand einer Figur näher erläutert ; es zeigt:

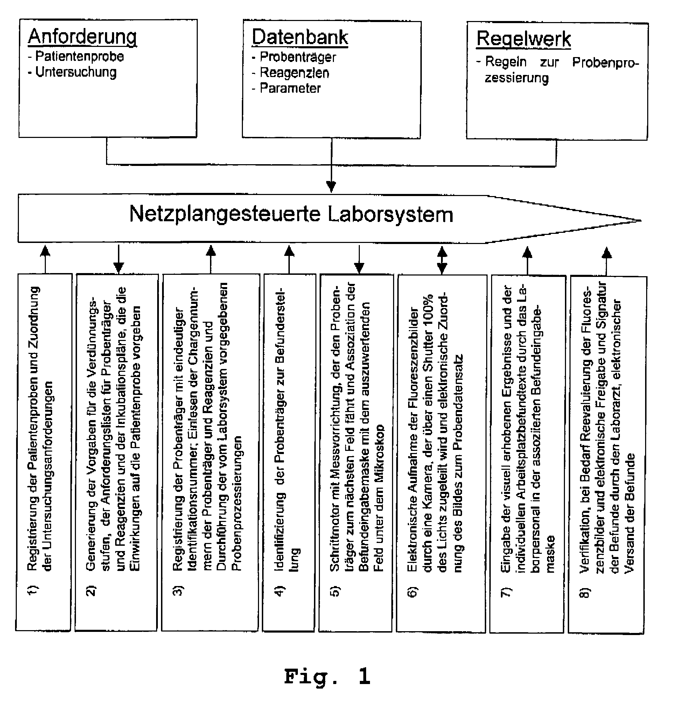

**Figur 1** ein Fließbild des erfindungsgemäßen Verfahrens.

Das in **Figur 1** dargestellte Fließbild, das auf einem netzplangesteuerten Laborsystem zur Erstellung von visuellen Patientenbefunden aufgrund manueller oder teilautomatisierter Analysen in der IIF beruht, zeigt im oberen Teil drei Blöcke, die mit "Anforderung", "Datenbank" und "Regelwerk" bezeichnet sind. Sie erläutern, welche Daten in das Laborsystem einzugeben sind bzw. welche Daten im Laborsystem hinterlegt sind.

Bei den "Anforderungen" sind es Daten über die Art der Patientenprobe wie Serum, Plasma, Liquor oder Vollblut, personenbezogene Daten wie Name, Geschlecht oder Geburtsdatum und die Art der Untersuchung auf Auto-Antikörper wie z.B. Antinukleäre Antikörper (ANA), auf Antikörper der Infektionsserologie wie z.B. Epstein-Barr-Virus (EBV) oder Antikörper der Allergie, die vom Laborpersonal zu einer eingegangenen Untersuchungsanforderung im Laborsystem eingegeben werden.

Der Block "Datenbank" erläutert, welche Stammdaten in einer zum Laborsystem zugehörigen Datenbank initial abgelegt werden müssen, damit das Laborsystem für die vom Labor grundsätzlich durchzuführenden Untersuchungen genutzt werden kann. Es handelt sich dabei im Wesentlichen um Stammdaten zu den Probenträgern, den Untersuchungsdefinitionen (Parametern) und den bei den Untersuchungen zu verwendenden Reagenzien. Die Daten zu einem Probenträger sind die Maße des Probenträgers, die Anzahl und Lage der Felder auf dem Probenträger, und die Art und die Position der Substrate auf den Feldern. Die Daten zu den Parametern umfassen sowohl das jeweils benötigte Substrat, bei dem es sich um aufgetragene Zellen, Gewebeschnitte oder biochemisch definierbare Substanzen handelt, die die Antigene enthalten, gegen die die Antikörper gerichtet sein können, als auch die Art und Menge der zu verwendenden Reagenzien. Die Daten über die Reagenzien beziehen sich auf die Konjugate, mit denen nachgewiesen wird, gegen welche Immunglubolinklasse der Antikörper gerichtet ist, die Verdünnungspuffer für die Patientenproben wie z. B. PBS-Tween, die testspezifischen Positivkontrollen und gegebenenfalls weitere Hilfsreagenzien.

Der Block "Regelwerk" umfasst die im Laborsystem hinterlegten Regeln zur Prozessierung der Proben. Das Regelwerk gibt u. a. vor, nach welchen Regeln die Auswahl der Probenträger vorgenommen wird und nach welchen Regeln die Probenprozessierungsschritte ermittelt werden, wie z.B. die Inkubationen der Probenträgerfelder mit verdünnten Patientenproben.

Im unteren Teil enthält das Fließbild Blöcke 1) bis 8), die verschiedene Verfahrensschritte angeben.

Zur Verarbeitung aller Eingaben und Verfahrensschritte dient das dazwischen liegende netzplangesteuerte Laborsystem.

Nachfolgend werden die einzelnen Blöcke näher erläutert:
Block 1):
   Dabei beginnt die Prozessierung der Patientenprobe mit dem Eingang der Patientenproben und der Begleitpapiere, die die Untersuchungsanforderungen enthalten. Die Proben und Begleitpapiere werden mit eindeutigen Identifikationsnummern (ID) gekennzeichnet, die im erfindungsgemäßen Laborsystem elektronisch registriert werden. Diese Proben-Identifikationsnummern werden im Laborsystem mit existierenden oder neu erfassten Patientendaten verknüpft und erzeugen Patientenprobendatensätze, denen die angeforderten Untersuchungen, die als Parameterstammdaten im Laborsystem hinterlegt sind, über eine standardisierte Maske im Laborsystem zugeordnet werden.
Block 2) :
   Das Laborsystem generiert zum einen die für die Durchführung der Untersuchungen erforderlichen Vorgaben für die Verdünnungsstufen der Proben und die Anforderungslisten für Reagenzien und Probenträger. Die Grundlage hierfür bilden die im Laborsystem als Stammdaten erfassten Probenträger und Reagenzien, deren Lagerbestände für jede Charge automatisch auf Basis der durchgeführten Untersuchungen aktualisiert werden. Das im Laborsystem konfigurierbar hinterlegte Regelwerk zur Erstellung der Anforderungslisten berücksichtigt neben den medizinischen Anforderungen auch eine kostenoptimale Verwendung der Probenträger, mit einer möglichst vollständigen Ausnutzung der verfügbaren Felderanzahl auf den Probenträgern. Zum anderen generiert das Laborsystem die Inkubationspläne, die Probendatensätze enthalten, die hinsichtlich Patientenproben und Probenträger eindeutig sind und eineindeutig mit den inkubierten Probenträgerfeldern assoziiert sind. Die Inkubationspläne geben alle Einwirkungen auf die Patientenprobe vor, so dass zu jeder Zeit abrufbar ist, welchen Prozessen die Patientenprobe zu unterwerfen ist bzw. unterworfen wurde.
Block 3):
   Das Laborpersonal oder ein vom Laborsystem angesteuerter Automat erstellt die Probenverdünnungen gemäß den vorgegebenen Verdünnungsstufen, kennzeichnet diese entsprechend eindeutig und liest diese elektronisch im Laborsystem ein. Anschließend entnimmt das Laborpersonal dem Lager die mit Chargennummern kodierten Probenträger und Reagenzien entsprechend den Anforderungslisten, kennzeichnet jeden entnommenen Probenträger mit einer eindeutigen Identifikationsnummer und registriert diese elektronisch im Laborsystem. Zusätzlich werden die Chargennummern der Probenträger und der Reagenzien im Laborsystem elektronisch eingelesen. Das Laborsystem verifiziert, dass für die angeforderten Untersuchungen die richtigen Probenträger und Reagenzien herangezogen werden. Vom Laborpersonal oder von einem vom Laborsystem angesteuerten Automaten werden die Einwirkungen auf die Patientenprobe gemäß den Inkubationsplänen vorgenommen: Im ersten Inkubationsschritt werden die registrierten Probenträger mit den Kontrollen und Probenverdünnungen inkubiert. Nach dem Waschvorgang werden die Probenträger nötigenfalls erneut im Laborsystem elektronisch identifiziert, um Verwechselungen von Probenträgern auszuschließen, und im zweiten Inkubationsschritt wird mit Fluoreszenzfarbstoffmarkierten Antikörpern inkubiert.
Block 4):
   Nachfolgend werden die Befunddaten zu den Patientenprobendaten durch Beurteilung des Fluoreszenzmusters am Mikroskop ermittelt. Im ersten Schritt der Ergebnisauswertung des erfindungsgemäßen Verfahrens wird der auszuwertende Probenträger über dessen Identifikationscode im Laborsystem elektronisch identifiziert.
Block 5):
   Gesteuert durch das Laborsystem, in dem die Maße der Probenträger konfigurierbar hinterlegt sind, fährt ein Schrittmotor mit Messvorrichtung den Probenträger, der in die Fassung des Mikroskoptisches unter dem Objektiv eingelegt ist, zum ersten bzw. mit Abschluss der Befundeingabe zum nächsten Probenträgerfeld, bzw. bei Feldern mit mehr als einem Substrat zum ersten Biochip des Feldes. Das Laborsystem assoziiert die Befundeingabemaske mit dem auszuwertenden Feld unter dem Mikroskop.
Block 6) :
   Jedes einzelne inkubierte Probenträgerfeld erzeugt für den mit diesem Feld eineindeutig assoziierten Probendatensatz individuelle Fluoreszenzbilder, die einem bestimmten Einzelbefund entsprechen. Die Position des Probenträgerfeldes unter dem Mikroskop kann in allen drei Achsen innerhalb eines Feldes vom Bedienpersonal eingestellt werden, wobei eine Autofokussierungseinheit den jeweils gewählten Bildausschnitt automatisch scharf stellt. Der Untersucher stellt einen oder mehrerer für die Auswertung relevante Bildausschnitte ein, nimmt diese mit einer elektronischen Kamera auf, das Laborsystem archiviert die aufgenommenen Fluoreszenzbilder und ordnet dieses dem Probendatensatz eineindeutig zu. Um optimale Lichtbedingungen für die Befunderhebung sowohl am Mikroskop als auch bei einer Reevaluierung auf Basis der aufgenommenen Fluoreszenzbilder zu gewährleisten, wird statt einer Teilung des Lichts zwischen Beobachter und Kamera, die häufig zu wenig Licht liefert, ein Shutter genutzt, der nur im Augenblick der Aufnahme 100% des Lichts der Kamera zuteilt. Im Gegensatz zu vollautomatischen Bildaufnahmegeräten kann der Laborarzt auf Basis dieser so erstellten relevanten Bildausschnitte eine Anhiebsdiagnose erheben.
Block 7):
   Sowohl die Ergebnisse, die z. B. über eine am Mikroskop befindliche Tastatur zum Probendatensatz eingegeben werden, als auch die individuelle Befunderhebung zu den Patientenprobendaten, bei der es möglich ist, patientenspezifische Befundtexte einzugeben, werden direkt am Mikroskoparbeitsplatz in der elektronisch assoziierten Befundeingabemaske zur Patientenprobe erstellt. Soll alternativ zur manuellen Prozessierung ein vollautomatisches Bildaufnahmegerät genutzt werden, das für sich alleine keine feste Zuordnung der Befunddaten zu den Patientenprobendaten vornehmen kann, kann dieses vom Laborsystem angesteuert werden, so dass bei der visuellen Befunderhebung der aufgenommenen Fluoreszenzbilder die Befundeingabemaske zur Patientenprobe vom Laborsystem mit dem aufgenommenen und auszuwertenden Bild assoziiert wird.
Block 8):
   Abschließend werden die individuellen Patientenbefunde mit den archivierten Fluoreszenzbildern und allen Kontrolluntersuchungen vom Laborarzt verifiziert. Dem Laborarzt wird eine authentische Kontrolle der Patientenbefunde ohne erneutes Mikroskopieren ermöglicht. Der Arzt müsste sonst den zumeist in einer Dunkelkammer befindlichen Probenträger heraussuchen, das Probenträgerfeld mit dem Befund assoziieren und das richtige Bild dem zu befundenden Patienten zuordnen. Diese umständlichen organzsatorzschen Maßnahmen entfallen und er kann die bereits korrekt zugeordneten Befunde an seinem Arbeitsplatz verifizieren, gegebenenfalls reevaluieren und anschließend elektronisch signieren und an den Einsender der Patientenprobe und Untersuchungsanforderung elektronisch versenden, z.B. per Fax oder verschlüsselter Mail. Zudem kann die Verifikation zeitversetzt ohne Qualitätsverlust erfolgen, während das erneute Mikroskopieren zeitnah erfolgen muss, da die gelagerten inkubierten Probenträger mit der Zeit an Qualität einbüßen.

## Patentansprüche

1. Netzplangesteuertes Verfahren zur Sicherstellung der Authentizität und Qualität visuell erhobener Befunde aufgrund manuell oder teilautomatisiert durchgeführter medizinischer Laboranalysen, mit der Technik "Indirekte Immunfluoreszenz" mit den Schritten:
a) Elektronisches Erfassen von Daten, die Patientenproben eindeutig identifizieren und elektronisches Zuordnen der Untersuchungsanforderungen;
b) elektronisches Generieren der Vorgaben für Verdünnungsstufen von Patientenproben und der Anforderungslisten für Reagenzien und Probenträger, auf denen Zellen, Gewebeschnitte oder biochemisch definierbare Substanzen aufgetragen sind;
c) elektronisches Generieren der Inkubationspläne, die die Patientenprobendaten den Probenträgerdaten zuordnen und Probendatensätze erzeugen, die hinsichtlich der Patientenproben und Probenträger eindeutig sind und die alle Einwirkungen auf die Patientenprobe vorgeben, so dass zu jeder Zeit abrufbar ist, welchen Prozessen die Patientenprobe zu unterwerfen ist bzw. unterworfen wurde;
d) elektronisches Erfassen der Daten von Probenträgern;
e) elektronisches Identifizieren der inkubierten und eindeutig über eine Identifikationsnummer gekennzeichneten Probenträger;
f) elektronisches Assoziieren der Befundeingabemaske zur Patientenprobe mit dem über eine Messvorrichtung exakt erfassten Probenträgerfeld unter dem Mikroskop;
g) elektronisch gesteuerte Aktualisierung des Probenträgerfeldes unter dem Mikroskop über einen Schrittmotor mit Messvorrichtung, der mit Abschluss der Befundeingabe den Probenträger unter dem Mikroskop zum nächsten Feld fährt;
h) elektronisch ausgelöste Aufnahme des Fluoreszenzbildes eines Probenträgerfeldes, bei dem über einen Shutter im Augenblick der Aufnahme 100% des Mikroskoplichts der Kamera zugeteilt wird;
i) elektronisches Archivieren der Fluoreszenzbilder und Zuordnen zu dem Probendatensatz, so dass zu jeder Zeit der erhobene Befund auf Basis dieser Bilder verifiziert werden kann;
j) elektronisches Eingeben aller visuell ermittelten Befunddaten in der assoziierten Befundeingabemaske;
k) elektronisches Signieren des Patientenbefundes; und
l) elektronisches Versenden des Patientenbefundes.
